# EUROPEAN PATENT SPECIFICATION

(11) **EP 2 594 993 B1**
(45) Date of publication and mention of the grant of the patent: **22.07.2015**
(21) Application number: 12192912.9
(22) Date of filing: 16.11.2012
(51) Int. Cl.: G03F 1/00, G03F 1/26, G03F 1/80, G06F 19/00, G06F 17/00

(54) **Evaluation of etch mask film**
Auswertung einer Ätzmaskenfolie
Évaluation de film de masque de gravure

(30) Priority: 18.11.2011 JP 2011252953
(43) Date of publication of application: 22.05.2013
(73) Proprietor: Shin-Etsu Chemical Co., Ltd., Chiyoda-ku Tokyo (JP)
(72) Inventor: Igarashi, Shinichi, Joetsu-shi, Niigata (JP); Yoshikawa, Hiroki, Joetsu-shi, Niigata (JP); Inazuki, Yukio, Joetsu-shi, Niigata (JP); Kaneko, Hideo, Joetsu-shi, Niigata (JP)
(74) Representative: Ter Meer Steinmeister & Partner

(56) References cited:
- EP-A2- 1 650 600
- WO-A1-2010/147172
- US-A1- 2009 253 054
- US-A1- 2011 229 807

## Description

### TECHNICAL FIELD

This invention relates to a method of evaluating an etch mask film of a photomask blank which is processed to form a photomask for use in the micro-fabrication of semiconductor integrated circuits, charge-coupled devices (CCD), liquid crystal display (LCD) color filters, and magnetic heads. More particularly, in conjunction with a photomask blank comprising a pattern-forming film and an etch mask film, the invention relates to a method of evaluating the etch mask film.

### BACKGROUND ART

In the recent semiconductor processing technology, a challenge to higher integration of large-scale integrated circuits places an increasing demand for miniaturization of circuit patterns. There are increasing demands for further reduction in size of circuit-constructing wiring patterns and for miniaturization of contact hole patterns for cell-constructing inter-layer connections. As a consequence, in the manufacture of circuit pattern-written photomasks for use in the photolithography of forming such wiring patterns and contact hole patterns, a technique capable of accurately writing finer circuit patterns is needed to meet the miniaturization demand.

In order to form a higher accuracy photomask pattern on a photomask substrate, it is of first priority to form a high accuracy resist pattern on a photomask blank. Since the photolithography carries out reduction projection in actually processing semiconductor substrates, the photomask pattern has a size of about 4 times the actually necessary pattern size, but an accuracy which is not loosened accordingly. The photomask serving as an original is rather required to have an accuracy which is higher than the pattern accuracy following exposure.

Further, in the currently prevailing lithography, a circuit pattern to be written has a size far smaller than the wavelength of light used. If a photomask pattern which is a mere 4-time magnification of the circuit feature is used, a shape corresponding to the photomask pattern is not transferred to the resist film due to influences such as optical interference occurring in the actual photolithography operation. To mitigate these influences, in some cases, the photomask pattern must be designed to a shape which is more complex than the actual circuit pattern, i.e., a shape to which the so-called optical proximity correction (OPC) is applied, or the photomask pattern must be designed while taking into account optical interference. Thus, at the present, the lithography technology for obtaining photomask patterns also requires a higher accuracy processing method. The lithographic performance is sometimes represented by a maximum resolution. As to the resolution limit, the lithography involved in the photomask processing step is required to have a maximum resolution accuracy which is equal to or greater than the resolution limit necessary for the photolithography used in a semiconductor processing step using a photomask.

A photomask pattern is generally formed by applying a photoresist film on a photomask blank having a light-shielding film on a transparent substrate, writing a pattern using electron beam, and developing to form a resist pattern. Using the resulting resist pattern as an etch mask, the light-shielding film is etched into a light-shield pattern. In an attempt to miniaturize the light-shield pattern, if processing is carried out while maintaining the thickness of the resist film at the same level as in the art prior to the miniaturization, the ratio of film thickness to pattern width, known as "aspect ratio," becomes higher. As a result, the resist pattern profile is degraded, preventing effective pattern transfer, and in some cases, the resist pattern can collapse or be stripped. Therefore, the thickness of resist film must be reduced to enable miniaturization.

As to the light-shielding film material to be etched through the resist pattern as etch mask, a number of materials are known in the art. Among others, chromium compound films are used in practice because many teachings about etching are available and their processing has been established as the standard process. For example, a photomask blank having a light-shielding film composed of a chromium compound suited for ArF excimer laser lithography is disclosed in JP-A 2003-195479. Specifically a chromium compound film having a thickness of 50 to 77 nm is described.

A typical dry etching process for chromium-based films such as chromium compound films is oxygen-containing chlorine base dry etching, which has a certain etching ability relative to organic film. Thus, when etching is conducted through a thinner resist film in order to transfer a finer size pattern for the above-described reason, the resist film can be damaged during etching. It is then difficult to transfer the resist pattern accurately. To meet both the requirements of miniaturization and accuracy, it becomes necessary to investigate the light-shielding material again so as to facilitate the processing of light-shielding film, rather than the current trend relying solely on resist performance improvement.

As to the light-shielding film material, silicon-based materials (e.g., materials containing silicon, or silicon and transition metal) allow for high accuracy processing as compared with the chromium-based materials used in the prior art. This is because the silicon-based materials have good light-shielding properties relative to exposure light of 200 nm or shorter, and can be processed by fluorine base dry etching which causes least damage to the resist pattern. See JP-A 2007-241065.

As to the technique of high accuracy processing using an etch mask, JP-A 2007-241060 discloses that the processing error associated with pattern dependency and side etching is reduced if a light-shielding film of silicon-based material is processed using a chromium-based material as the etch mask. Then, a light-shielding film of silicon-based material to be combined with an etch mask film of chromium-based material is regarded promising as the light-shielding material of the next generation.

### Citation List

- Patent Document 1:: JP-A 2003-195479
- Patent Document 2:: JP-A 2007-241065
- Patent Document 3:: JP-A 2007-241060
(US 20070212619, EP 1832926)

### SUMMARY OF INVENTION

To form a photomask pattern having a feature size of 50 nm or less, a resist film having a thickness of 150 nm or less is required. The etch mask film optimized for such a thin resist film should have a thickness of 30 nm or less. On use of such etch mask film, a thinner etch mask film is desirable because the resist film can also be thin. However, if the etch mask film is chosen only from the aspect of thinning, a problem arises that the pattern of the pattern-forming film contains defects or the pattern is not formed to the design. Also, the etch mask film is preferably resistant to the etching of the pattern-forming film. However, the film which is resistant to the etching of the pattern-forming film is often resistant to the etching of the etch mask film as well. Then the resist film intended for patterning of the etch mask film cannot be thin.

The etch mask film must have at least a sufficient thickness to ensure that the film is left until the etching of the pattern-forming film is complete. On the other hand, the etch mask film should be as thin as possible so that it may be patterned without causing substantial damages to the resist film.

An object of the invention is to provide a method of evaluating an etch mask film, which enables to evaluate both the etch resistance during etching of the pattern-forming film and the etchability during etching of the etch mask film itself whereby an etch mask film having satisfactory etching performance can be found.

Studying the etching behavior of an etch mask film, the inventors found that during etching of a pattern-forming film, the etch mask film is etched independent of the identity of material of which it is made, although the etching clear time is long.

Additionally it was found that defects are introduced in the pattern of the pattern-forming film, typically light-shielding film, because the resist film serving as an etch mask during patterning of the etch mask film can be etched during patterning of the etch mask film, and also because the etch mask film serving as an etch mask during patterning of the pattern-forming film can be etched during patterning of the pattern-forming film.

The invention pertains to a photomask blank comprising a transparent substrate, a pattern-forming film on the substrate for forming a photomask pattern, and an etch mask film on the pattern-forming film for serving as a mask during etching of the pattern-forming film. It is intended herein that for the etch mask film, both its etch resistance during etching of the pattern-forming film and its etchability during etching of the etch mask film itself are evaluated in a relative manner. It has been found that the performance of the etch mask film can be evaluated by measuring a first etching clear time (C1) taken when the etch mask film is etched under the etching conditions to be applied to the pattern-forming film when a photomask pattern is formed from the pattern-forming film, measuring a second etching clear time (C2) taken when the etch mask film is etched under the etching conditions to be applied to the etch mask film when an etch mask pattern is formed from the etch mask film, and computing a ratio (C1/C2) of the first to second etching clear time. Then an etch mask film affording appropriate etching performance can be selected.

In conjunction with a photomask blank comprising a transparent substrate, a pattern-forming film on the substrate for forming a photomask pattern, and an etch mask film on the pattern-forming film for serving as a mask during etching of the pattern-forming film, the invention provides a method of evaluating the etch mask film comprising the steps of:
measuring a first etching clear time (C1) taken when the etch mask film is etched under the etching conditions to be applied to the pattern-forming film when a photomask pattern is formed from the pattern-forming film,
measuring a second etching clear time (C2) taken when the etch mask film is etched under the etching conditions to be applied to the etch mask film when an etch mask pattern is formed from the etch mask film, and
computing a ratio (C1/C2) of the first to second etching clear time, by which the performance of the etch mask film is evaluated.

In a preferred embodiment, the etching to be applied to the pattern-forming film is fluorine base dry etching, and the etching to be applied to the etch mask film is chlorine base dry etching.

In a preferred embodiment, the pattern-forming film is formed of a material comprising silicon and another metal, and the etch mask film is formed of a silicon-free material comprising chromium.

### ADVANTAGEOUS EFFECTS OF INVENTION

Using the evaluation method, an etch mask film having appropriate etching performance can be selected. When a photomask is prepared from a photomask blank having such an appropriate etch mask film, the transfer of the photomask pattern to the pattern-forming film is satisfactory and the resulting photomask has minimal pattern defects.

### BRIEF DESCRIPTION OF DRAWINGS

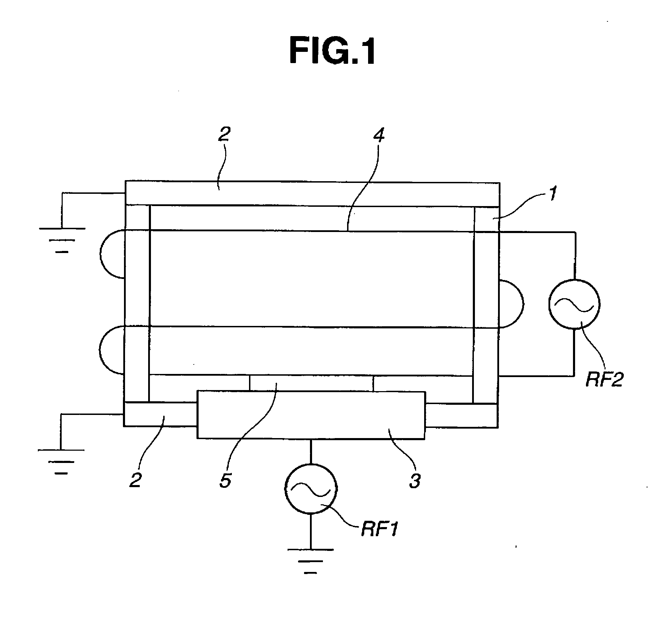

The only figure, FIG. 1 schematically illustrates a dry etching system used in Example.

### DESCRIPTION OF PREFERRED EMBODIMENTS

The terms "first", "second", and the like, as used herein do not denote any order, quantity, or importance, but rather are used to distinguish one element from another.

The invention pertains to a photomask blank comprising a transparent substrate such as quartz substrate, a pattern-forming film deposited on the substrate for forming a photomask pattern, and an etch mask film deposited on the pattern-forming film and serving as a mask during etching of the pattern-forming film. The method of the invention is to evaluate the etching performance of the etch mask film formed on the pattern-forming film, preferably contiguous to the pattern-forming film.

Examples of the pattern-forming film include a light-shielding film adapted to form a light-shielding pattern upon fabrication of a photomask, and a phase shift film such as a halftone phase shift film adapted to form a phase shift pattern. That is, the pattern-forming film is a functional film affording an optical function as a photomask pattern when a photomask resulting from the photomask blank is used. On the other hand, the etch mask film is a film which exhibits etch resistance when the pattern-forming film is etched. The etch mask film is also known as "hard mask film" in that the film is used as the etch mask pattern when the pattern-forming film is etched. The pattern-forming film and etch mask film each may be a film of a single layer or multiple layers. The pattern-forming film may include an antireflective layer, adhesion-improving layer, protective layer or the like.

The pattern-forming film and etch mask film may have different etching performance. For example, one of the pattern-forming film and etch mask film, especially the pattern-forming film may be a film which is susceptible to fluorine base dry etching with fluorine base etchant gas such as SF₆ or CF₄, and less susceptible to chlorine base dry etching with chlorine base etchant gas containing chlorine and oxygen gases. Suitable such films include, for example, a silicon film, a film containing silicon, but not metals other than silicon, and a film containing silicon and another metal(s). Examples of the metals other than silicon include molybdenum, tungsten, tantalum and zirconium, and mixtures thereof. Of these, molybdenum is preferred for workability. These films may further contain one or more light elements selected from among oxygen, nitrogen, carbon and hydrogen.

The other film (which is an etch mask film if the one film is a pattern-forming film) may be a film which is less susceptible to fluorine base dry etching with fluorine base etchant gas such as SF₆ or CF₄, and susceptible to chlorine base dry etching with chlorine base etchant gas containing chlorine and oxygen gases. Suitable such films include, for example, a film of a silicon-free material containing chromium and a film of a silicon-free material containing tantalum. These films may further contain one or more light elements selected from among oxygen, nitrogen, carbon and hydrogen. It is noted that both the chlorine base etchant gas and the fluorine base etchant gas may further contain an inert gas such as helium or argon.

The combination of etchings is exemplified above by a combination of fluorine base dry etching using fluorine base etchant gas with chlorine base dry etching using chlorine base etchant gas containing chlorine gas and oxygen gas, but not limited thereto. Other combinations include, but are not limited to,
1) a combination of chlorine base dry etching using chlorine base etchant gas containing chlorine gas, but not oxygen gas with chlorine base dry etching using chlorine base etchant gas containing chlorine gas and oxygen gas;
2) a combination of two chlorine base dry etchings using chlorine base etchant gases containing chlorine gas and oxygen gas in a different ratio; and
3) a combination of fluorine base dry etching using fluorine base etchant gas with chlorine base dry etching using chlorine base etchant gas containing chlorine gas, but not oxygen gas.

The method of forming a photomask pattern from a pattern-forming film (typically light-shielding film) using an etch mask film is described by referring to one embodiment wherein a photomask is prepared from a photomask blank in which the pattern-forming film is a MoSi-based light-shielding film and the etch mask film is a chromium-based film.

First of all, a photomask blank is prepared by depositing a MoSi-based light-shielding film and a chromium-based etch mask film on a substrate in sequence. A resist film is formed on the chromium-based etch mask film. The resist film is processed to form a resist pattern, after which the etch mask film is patterned by chlorine base dry etching. Then the resist film is stripped off. While the etch mask film serves as an etch mask, the light-shielding film is patterned by fluorine base dry etching, whereby a photomask is obtained. If desired, the etch mask pattern may be removed.

To form a fine size photomask pattern at a high accuracy, a thinner resist film is recommended from the standpoint of aspect ratio. The thickness of a resist film depends on the etching time taken when the etch mask film is patterned. In order that a thin resist film allow for satisfactory patterning, the etching time of the etch mask film must be short. Satisfactory patterning within a short etching time is achievable by (1) designing the composition and construction of the etch mask film for a short etching clear time and/or (2) reducing the thickness of the etch mask film.

However, when the pattern-forming film such as a light-shielding film is patterned using the etch mask film, the etch mask film must be fully resistant to the etching of the pattern-forming film. An improvement in etch resistance is achievable by (3) designing the composition and construction of the etch mask film for a long etching clear time and/or (4) increasing the thickness of the etch mask film.

Namely, it is required to the etch mask film that the film composition and construction be designed so as to provide for a short etching clear time under the etching conditions for the etch mask film and a long etching clear time under the etching conditions for the pattern-forming film.

According to the invention, the performance of an etch mask film is evaluated by measuring a first etching clear time (C1) taken when the etch mask film is etched under the etching conditions to be applied to the pattern-forming film when a photomask pattern is formed from the pattern-forming film, measuring a second etching clear time (C2) taken when the etch mask film is etched under the etching conditions to be applied to the etch mask film when an etch mask pattern is formed from the etch mask film, and computing a ratio (C1/C2) of the first to second etching clear time.

The method of the invention is an evaluation method in which the clear time ratio C1/C2 of first etching clear time C1 taken when the etch mask film is etched under the etching conditions to be applied to the pattern-forming film to second etching clear time C2 taken when the etch mask film is etched under the etching conditions to be applied to the etch mask film is utilized as a figure of merit (FOM), that is, the clear time ratio C1/C2 is an index for the etching behavior of an etch mask film of a certain composition and construction.

The first etching clear time C1 corresponds to etch resistance of the etch mask film during etching of the pattern-forming film whereas the second etching clear time C2 corresponds to etchability during etching of the etch mask film itself. Accordingly, the higher the clear time ratio C1/C2 is, the better the film performs. The clear time ratio C1/C2 is preferably at least 8, more preferably at least 9, and even more preferably at least 9.5. The upper limit of the ratio C1/C2 is not critical. However, in a typical embodiment wherein the etch mask film is a chromium-based film, the etchant gas applied to the pattern-forming film is fluorine base gas, and the etchant gas applied to the etch mask film is chlorine base gas, for example, the ratio C1/C2 is up to 12 in the practical application.

If an etch mask film having a higher clear time ratio C1/C2 is used, then both the quantity of the resist film etched during etching of the etch mask film and the quantity of the etch mask film etched during etching of the pattern-forming film can be relatively minimized.

The "etching clear time" refers to a time duration when etching of a film having a certain thickness is cleared, that is, a time passed from the start of etching until the underlying layer or substrate is exposed when the film is removed. The etching clear time may be determined by measuring the reflectivity of a film during etching and detecting a change of reflectivity, from which the completion of etching is judged, or if a film is visually observable during etching, by visually observing and confirming the exposure of the underlying layer or substrate, from which the completion of etching is judged. Alternatively, the etching clear time may be determined by analyzing the emission spectrum of a plasma in the etching chamber, analyzing a certain ion or element in the plasma, and detecting a spectral change, from which the completion of etching is judged.

The thickness of the etch mask film may be determined in terms of the etching clear times of the etch mask film and pattern-forming film under the etching conditions for the pattern-forming film. It suffices that the etching clear time of the etch mask film is longer than the etching clear time of the pattern-forming film. The thickness of the etch mask film is determined such that the etching clear time of the etch mask film may be longer than the etching clear time of the pattern-forming film by a factor of 1.1 to 5, especially 1.2 to 3. As to the thickness of the etch mask film, a thinner film is preferred. However, too thin a film may allow for pattern defects whereas too thick a film may require a thicker resist film which is disadvantageous for fine size pattern formation and leads to larger pattern density dependence or optical proximity effect. For this reason, the thickness of the etch mask film is preferably in a range of 1 to 30 nm, more preferably 1 to 10 nm. The thickness of the resist film which is applied to the etch mask film within the above thickness range is preferably in a range of 30 to 200 nm, more preferably 30 to 150 nm, and even more preferably 30 to 100 nm. On the other hand, the thickness of the pattern-forming film, which varies with its optical function, is typically 10 to 100 nm, preferably 20 to 80 nm, relative to the etch mask film within the above thickness range.

The method of evaluating an etch mask film according to the invention is effective for evaluating the relevant film in a binary photomask blank comprising a transparent substrate, a light-shielding film formed as the pattern-forming film on the substrate, and an etch mask film formed thereon, and a phase shift mask blank comprising a transparent substrate, a phase shift film (typically halftone phase shift film) formed as the pattern-forming film on the substrate, an optional light-shielding film formed thereon, and an etch mask film formed thereon.

### EXAMPLE

Examples of the invention are given below by way of illustration and not by way of limitation.

### Example 1

Photomask blank samples were prepared by depositing a light-shielding film of MoSi-based material on a quartz substrate, and depositing a film of chromium-based material thereon as an etch mask film. For deposition of the chromium-based material film, the target used was metallic chromium and the sputtering gas was a mixture of argon, nitrogen and oxygen. A single layer film and multilayer films of the chromium-based material, total four films, were deposited on the light-shielding film while the deposition conditions were varied. In this way, four photomask blank samples A to D were prepared. The deposition time was adjusted such that the etch mask film had a thickness of 3 nm.

The chromium-based material films thus deposited were determined for etching clear times of chlorine base dry etching and fluorine base dry etching. The outline of a dry etching system used in these etching tests is illustrated in FIG. 1. The system includes a chamber 1, grounded plates 2, a lower electrode 3, an antenna coil 4, a substrate 5 to be treated, and radio frequency power supplies RF1 and RF2.

The chlorine base dry etching test was under the following conditions.
RF1 (RIE): pulse 700 V
RF2 (ICP): CW 400 W
Pressure: 6 mTorr
Cl₂: 185 sccm
O₂: 55 sccm
He: 9.25 sccm

It is noted that RIE stands for reactive ion etching, ICP for inductively coupled plasma, and CW for continuous wave oscillation. During the etching test, the intensity of plasma emission (wavelength 520 nm) attributable to chlorine atom was monitored to determine a change of intensity with time. For four photomask blank samples, the etching clear time C2 of the etch mask film is shown in Table 1.

The fluorine dry etching test was under the following conditions.
RF1 (RIE): CW 54 V
RF2 (ICP): CW 325 W
Pressure: 5 mTorr
SF₆: 18 sccm
O₂: 45 sccm

During the etching test, the reflectivity is monitored using inspection light of wavelength 675 nm, to determine a change of reflectivity with time. Once the chromium-based material film is completely etched, etching of the light-shielding film of MoSi-based material starts. As the light-shielding film loses its thickness, the reflectivity decreases and eventually reaches the value of the reflectivity of the quartz substrate. For four photomask blank samples, the etching clear time C1 of the etch mask film is shown in Table 1.

**Table 1**

| Sample | Etching clear time (sec) | | C1/C2 |
|---|---|---|---|
| | Chlorine C2 | Fluorine, C1 | |
| A | 16.5 | 159 | 9.6 |
| B | 15.6 | 148 | 9.5 |
| C | 12.8 | 116 | 9.1 |
| D | 13.7 | 103 | 7.5 |

A clear time ratio C1/C2 was computed by dividing etching clear time C1 for fluorine base dry etching by etching clear time C2 for chlorine base dry etching. For all the samples, the film thickness was identical. Among samples A to D, sample A shows the highest ratio C1/C2, indicating a film of the best composition and construction.

## Claims

1. A method of evaluating an etch mask film comprising the steps of:
providing a photomask blank comprising a transparent substrate, a pattern-forming film on the substrate for forming a photomask pattern, and an etch mask film on the pattern-forming film for serving as a mask during etching of the pattern-forming film,
measuring a first etching clear time (C1) taken when the etch mask film is etched under the etching conditions to be applied to the pattern-forming film when a photomask pattern is formed from the pattern-forming film,
measuring a second etching clear time (C2) taken when the etch mask film is etched under the etching conditions to be applied to the etch mask film when an etch mask pattern is formed from the etch mask film, and
computing a ratio (C1/C2) of the first to second etching clear time, by which the performance of the etch mask film is evaluated.

2. The evaluating method of claim 1 wherein the etching to be applied to the pattern-forming film is fluorine base dry etching, and the etching to be applied to the etch mask film is chlorine base dry etching.

3. The evaluating method of claim 1 or 2 wherein the pattern-forming film is formed of a material comprising silicon and another metal, and the etch mask film is formed of a silicon-free material comprising chromium.

## Patentansprüche

1. Verfahren zur Bewertung einer Ätzmaskenfolie, umfassend die Schritte:
Vorsehen eines Fotomaskenrohlings, umfassend ein transparentes Substrat, einen musterbildenden Film auf dem Substrat zur Bildung eines Fotomaskenmusters, und eine Ätzmaskenfolie auf dem musterbildenden Film, um als Maske während dem Ätzen des musterbildenden Films zu dienen,
Messen einer ersten Ätzklarzeit (C1), die bestimmt wird, wenn die Ätzmaskenfolie geätzt wird, unter den Ätzbedingungen, die für den musterbildenden Film anzuwenden sind, wenn ein Fotomaskenmuster aus dem musterbildenden Film gebildet wird,
Messen einer zweiten Ätzklarzeit (C2), die bestimmt wird, wenn die Ätzmaskenfolie geätzt wird, unter den Ätzbedingungen, die für die Ätzmaskenfolie anzuwenden sind, wenn ein Ätzmaskenmuster aus der Ätzmaskenfolie gebildet wird, und
Berechnen eines Verhältnisses (C1/C2) aus der ersten zu der zweiten Ätzklarzeit, durch welches die Leistungsfähigkeit der Ätzmaskenfolie bewertet wird.

2. Bewertungsverfahren nach Anspruch 1, wobei das Ätzen, das für den musterbildenden Film anzuwenden ist, Trockenätzen auf Fluorbasis ist, und das Ätzen, das für die Ätzmaskenfolie anzuwenden ist, Trockenätzen auf Chlorbasis ist.

3. Bewertungsverfahren nach Anspruch 1 oder 2, wobei der musterbildende Film aus einem Material gebildet ist, umfassend Silizium und ein weiteres Metall, und die Ätzmaskenfolie aus einem siliziumfreien, Chrom umfassenden Material gebildet ist.

## Revendications

1. Un procédé d'évaluation d'une couche de masque de gravure, comprenant les étapes de:
la mise à disposition d'un photomasque vierge comprenant un support transparent, une couche de formation de motif sur le support pour la formation d'un motif de photomasque, et une couche de masque de gravure sur la couche de formation de motif afin de servir en tant que masque lors de la gravure de la couche de formation de motif,
la mesure d'un premier temps du graver transparent (C1) pris lorsque la couche de masque de gravure est gravée dans les conditions de gravure devant être appliquée à la couche de formation de motif quand un motif de photomasque est formé à partir de la couche de formation de motif,
la mesure d'un deuxième temps du graver transparent (C2) pris lorsque la couche de masque de gravure est gravée dans les conditions de gravure devant être appliquée à la couche de masque de gravure quand un motif de masque de gravure est formé à partir de la couche de masque de gravure, et
le calcul d'un rapport (C1 / C2) du premier au deuxième temps du graver transparent, par lequel la performance de la couche de masque de gravure est évaluée.

2. Le procédé d'évaluation de la revendication 1, la gravure devant être appliquée à la couche de formation de motif étant la gravure à sec a la base de fluor, et la gravure devant être appliquée à la couche de masque de gravure étant la gravure à sec a la base de chlore.

3. Le procédé d'évaluation de la revendication 1 ou de la revendication 2, la couche de formation de motif étant formée d'un matériau comprenant du silicium et un autre métal, et la couche de masque de gravure étant formé d'un matériau exempt de silicium comprenant du chrome.
